# FASCICULE DE BREVET EUROPEEN

(11) **EP 1 464 330 B1**
(45) Date de publication et mention de la délivrance du brevet: **16.05.2007**
(21) Numéro de dépôt: 04075969.8
(22) Date de dépôt: 20.12.2000
(51) Int. Cl.: A61Q 19/00, A61K 8/60, A61K 8/44, A61K 31/702, A61P 17/00, A61P 37/08, A61P 29/00

(54) **composition antiallergique comprenant des oligosaccharides et une saponine ou un acide aminé basique destinée à être administrée par voie topique ou par inhalation**
Antiallergische Zusammensetzung, die OLIGOSACCHARIDe und eine Saponine oder eine basische Aminosäure enthält, zur topischen Verabreichung oder Inhalation
antiallergic composition containing oligosaccharides in combination with a saponine or a basic aminoacid, for topical administration or for inhalation

(30) Priorité: 20.12.1999 FR 9916060
(43) Date de publication de la demande: 06.10.2004
(62) Demande divisionnaire de: 00990076.2
(73) Titulaire: Laboratoires G Pharm, 34980 St. Clément de Rivière (FR)
(72) Inventeur: Francisco, Christian, 66250 Saint Laurent de la Salanque (FR); Martinez, Gérard, 34980 Saint Clement de Rivière (FR)
(74) Mandataire: Warcoin, Jacques

(56) Documents cités:
- EP-A- 0 652 012

## Description

La présente invention concerne une composition, notamment topique externe cosmétique ou dermatologique, contenant des oligosaccharides, et une saponine ou un acide aminé basique, à une concentation comprise entre 5% et 20% en poids par rapport à la concentration en oligosaccharides.

Le domaine de la présente invention est celui des symptômes désagréables associés ou non à l'allergie et en particulier à l'asthme, l'eczéma, le prurit, le psoriasis, la conjonctivite allergique, l'urticaire, les réactions aux piqûres d'insectes, les démangeaisons en particulier les démangeaisons symptomatiques des grands brûlés.

Les propriétés de dégranulation des polynucléaires et notamment des éosinophiles, basophiles et mastocytes sont connues pour être impliquées dans les phénomènes d'allergies.

Ainsi, les basophiles matures du sang présentent des granules distribués au hasard et bordés par une membrane, ces granules contiennent divers produits (héparine, SRS-A, ECF-A) qui sont libérés lorsqu'un stimulus approprié induit une dégranulation. Ce stimulus est habituellement un allergène qui apparie les lgE spécifiques fixées à la surface de la cellule par les récepteurs appropriés. Les produits libérés par la dégranulation sont responsables d'une partie des symptômes désagréables associés à l'allergie, mais ils sont également impliqués dans l'immunité anti-parasitaire.

EP 652 012 décrit un très grand nombre de combinaisons sucre + acide aminé pour le traitement de différentes conditions mais ne décrit pas spécifiquement la combinaison raffinose + arginine dans les proportions revendiquées. En outre les combinaisons sucre + acide aminé de EP 652 012 sont destinées à être ingérées, et non à être administrées par voie topique externe ou par inhalation.

Les principaux composés de l'art antérieur connus pour leurs propriétés anti-allergiques sont les composés de la famille des anti-histaminiques telle que la chlorpheniramine (polaramine). Ces composés ne sont pas dépourvus d'effets secondaires tels que des risques de somnolence. C'est pourquoi la présente invention propose l'utilisation d'une association de composés qui sont dépourvus des effets secondaires des anti-histaminiques classiques et qui permettent néanmoins de traiter les allergies et autres affections ou désagréments du même type.

La Demanderesse a mis en évidence que l'utilisation de certains oligosaccharides est efficace pour réduire ou même bloquer les symptômes désagréables associés ou non à l'allergie et en tant qu'anti-inflammatoire.

Sans vouloir être liée à une quelconque théorie, il apparaît que la Demanderesse a montré que certains oligosaccharides possèdent une activité anti-dégranulante, anti-granulante ou anti-activatrice d'une cellule cible de l'allergie et notamment du basophile humain stimulé selon une réaction IgE dépendante.

D'autres cellules spécialisées de la peau (comme par exemple les neurones qui par libération de substance P sont responsables de la douleur mais aussi induisent une dégranulation des basophiles) sont aussi concernés par cette activité.

Le phénomène de dégranulation ainsi que les symptômes désagréables qui y sont liés sont connus de l'homme du métier cependant les inventeurs de la présente demande n'excluent pas que l'association utilisée au sens de la présente demande intervienne en amont de la dégranulation : lors de la maturation des granules. C'est pourquoi, selon les inventeurs, cette association pourrait aussi posséder une activité dite « anti-granulante ».

La présente invention concerne plus particulièrement une association comprenant au moins deux composants:
un premier composant qui est un oligosaccharide de formule I contenant de trois à cinq unités osidiques et possédant au moins une unité D-galactose liée par une liaison α[1-6] avec une unité sucrose : 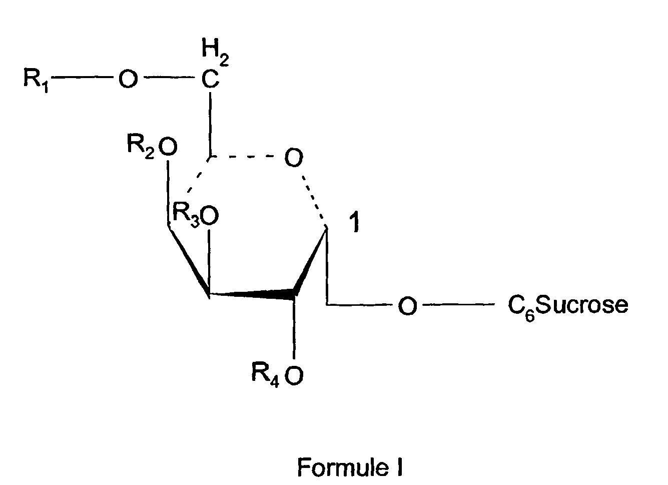 dans laquelle R₁, R₂, R₃ et R₄, indépendamment les uns des autres représentent un atome d'hydrogène, un groupe alkyle contenant de 1 à 10 atomes de carbone et possédant éventuellement au moins une insaturation, une fonction acide contenant de 1 à 10 atomes de carbone et possédant éventuellement au moins une insaturation, une fonction sulfate ou encore un ose,
et au moins un deuxième composant qui est une molécule chargée positivement au pH physiologique et favorisant la pinocytose et/ou une molécule favorisant la pénétration membranaire.
   Le deuxième composant agit comme vecteur du premier composant, ce premier composant étant le principe actif. Ce deuxième composant a pour rôle essentiel de permettre au premier composant, le principe actif, de franchir la membrane des globules blancs.

Ledit oligosaccharide peut en particulier être choisi dans le groupe constitué par le raffinose et le stachyose sous forme libre ou dérivée. Le deuxième composant est une saponine ou un acide aminé basique, de manière préférée, il est choisi dans le groupe constitué par les saponines et l'arginine.

De manière préférée, les saponines naturelles suivantes seront utilisées : les harpagosides, les ginsenosides, les saponines de type quillaja saponaria QS III et QS 21A.

La présente invention a trait à une composition cosmétique pour inhiber la granulation et/ou la dégranulation des globules blancs destinée à être administrée par voie topique externe ou par inhalation telle que le premier composant - le principe actif- est un oligosaccharide de formule I contenant de trois à cinq unités osidiques et possédant au moins une unité D-galactose liée par une liaison α[1-6] avec une unité sucrose : 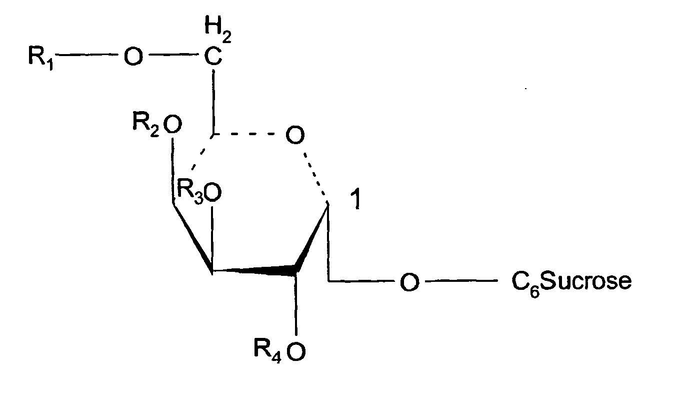 dans laquelle R₁, R₂, R₃ et R₄, indépendamment les uns des autres représentent un atome d'hydrogène, un groupe alkyle contenant de 1 à 10 atomes de carbone et possédant éventuellement au moins une insaturation,une fonction acide contentant de 1 à 10 atomes de carbone et possédant éventuellement au moins une insaturation, une fonction sulfate ou encore un ose, ledit oligosaccharide étant de préférence choisi dans le groupe constitué par le raffinose et le stachyose sous forme libre ou dérivée, et
le deuxième composant est une molécule chargée positivement au pH physiologique et favorisant la pinocytose et/ou une molécule favorisant la pénétration membranaire le deuxième composant est une saponine ou un acide aminé basique.

Ladite composition cosmétique peut encore contenir un composant anti-allergique.

Les associations préférées pour une composition selon la présente invention sont les suivantes : l'oligosaccharide est le raffinose sous forme libre ou dérivée et le deuxième composant une saponine ; l'oligosaccharide est le raffinose sous forme libre ou dérivée et le deuxième composant est l'arginine ; l'oligosaccharide est le stachyose sous forme libre ou dérivée et le deuxième composant est une saponine ; l'oligosaccharide est le stachyose sous forme libre ou dérivée et le deuxième composant est l'arginine.

Selon une forme préférée, ladite composition est telle que la concentration pondérale en oligosaccharide est comprise entre 0,01 % et 20 % par rapport à la masse totale de la composition, de préférence entre 0,01 % et 10 % par rapport à la masse totale de la composition. La concentration du deuxième composant (saponines, arginine) est comprise entre 5 et 20 % en poids par rapport à la concentration en oligosaccharide, de préférence environ 10 % en poids par rapport à la concentration en oligosaccharide.
Cette composition contient en outre généralement au moins un excipient cosmétiquement acceptable.

Les compositions cosmétiques utilisées selon la présente invention peuvent se présenter sous forme d'une solution, émulsion, crème, pommade, poudre, lait, lotion, gel ou pâte à l'eau. Sans vouloir être lié par une quelconque théorie, il semblerait que l'ingestion (boissons, tablettes, gélules) rende la composition inefficace, car vraisemblablement, les systèmes enzymatiques de l'estomac détruisent la partie sucrose de la molécule, la transformant en mélibiose. Or, les études *in vitro* ont montré que le mélibiose n'était pas actif sur les leucocytes.

La présente invention a en outre pour objet une composition dermatologique, c'est à dire une composition à titre de médicament, pour inhiber la granulation et/ou la dégranulation des globules blancs, destinée à être utilisée par voie topique externe ou par inhalation. Cette composition est notamment destinée au traitement d'au moins un symptôme associé ou non à l'allergie choisi dans le groupe constitué par l'asthme, l'eczéma, le prurit, le psoriasis, la conjonctivite allergique, l'urticaire, les réactions aux piqûres d'insectes, les démangeaisons en particulier les démangeaisons symptomatiques des grands brûlés ou en tant qu'anti-inflammatoire en particulier pour le traitement de l'arthrose.

La présente invention concerne l'utilisation pour la fabrication d'un médicament pour inhiber la granulation et/ou la dégranulation des globules blancs destinée à être prise sous forme de pommades, crèmes, collyres, aérosols (sprays), lotions ou par inhalation d'une composition comprenant au moins :
Le premier composant -le principe actif- de ladite association est un oligosaccharide de formule 1 contenant de trois à cinq unités osidiques et possédant au moins une unité D-galactose liée par une liaison α[1-6] avec une unité sucrose : 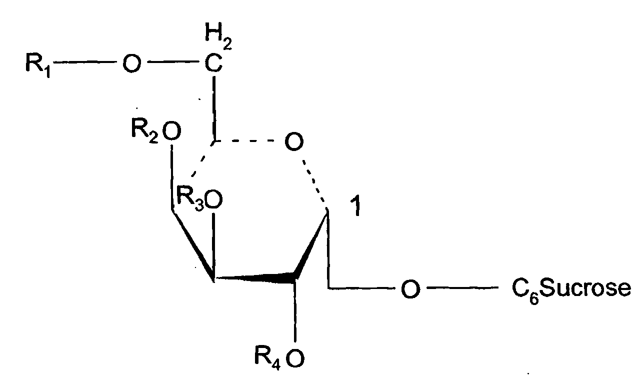 dans laquelle R₁, R₂, R₃ et R₄, indépendamment les uns des autres représentent un atome d'hydrogène, un groupe alkyle contenant de 1 à 10 atomes de carbone et possédant éventuellement au moins une insaturation, une fonction acide contenant de 1 à 10 atomes de carbone et possédant éventuellement au moins une insaturation, une fonction sulfate ou encore un ose , ledit oligosaccharide étant de préférence choisi dans le groupe constitué par le raffinose et le stachyose sous forme libre ou dérivée, et le
deuxième composant est une molécule chargée positivement au pH physiologique et favorisant la pinocytose ou une molécule favorisant la pénétration membranaire. Le deuxième composant est une saponine ou un acide aminé basique, de manière préférentielle il est choisi dans le groupe constitué par les saponines et l'arginine.
Les associations préférées sont les suivantes : l'oligosaccharide est le raffinose sous forme libre ou dérivée et le deuxième composant une saponine; l'oligosaccharide est le raffinose sous forme libre ou dérivée et le deuxième composant est l'arginine ; l'oligosaccharide est le stachyose sous forme libre ou dérivée et le deuxième composant est une saponine ; l'oligosaccharide est le stachyose sous forme libre ou dérivée et le deuxième composant est l'arginine.

Selon une forme préférée, ladite composition est telle que la concentration pondérale en oligosaccharide est comprise entre 0,01 % et 20 % par rapport à la masse totale de la composition, de préférence entre , 0,01 % et 10 % par rapport à la masse totale de la composition. La concentration du deuxième composant (saponines, arginine ) est comprise entre 5 et 20% en poids par rapport à la concentration en oligosaccharide, de préférence environ 10% en poids par rapport à la concentration en oligosaccharide.
Ladite composition à titre de médicament peut encore contenir un composant anti-allergique.
Ledit médicament contient avantageusement au moins un excipient
pharmaceutiquement acceptable.

Ce médicament peut être destiné à un traitement local ou général, et peut se présenter sous la forme d'une solution, émulsion, crème, pommade, poudre, lait, lotion, gel ou pâte à l'eau, collyre, spray.

Les principales indications de ce médicament sont les traitements d'au moins un symptôme associé ou non à l'allergie choisi dans le groupe constitué par l'asthme, l'eczéma, le prurit, le psoriasis, la conjonctivite allergique, l'urticaire, les réactions aux piqûres d'insectes ou les démangeaisons en particulier les démangeaisons symptomatiques des grands brûlés, ce médicament est en outre préconisé en tant qu'anti-inflammatoire, en particulier pour le traitement de l'arthrose.

La présente invention a encore pour objet un procédé de préparation d'une composition selon l'invention telle que l'on mélange au moins :
un oligosaccharide de formule I,
un deuxième composant qui est une molécule chargée positivement au pH physiologique et favorisant la pinocytose ou une molécule favorisant la pénétration membranaire, choisi parmi une saponine et un acide aminé basique éventuellement un composant anti-allergique.

Ces oligosaccharides constitués d'unités galactose reliées par une (ou des) liaisons de type α entre elles et à une unité sucrose, sont les substances de réserve d'un certain nombre de plantes (haricots, pois, soja,...) et sont surtout connus pour leur responsabilité dans le phénomène de flatulence chez l'homme. Par contre, aucune action directe pouvant présenter un intérêt thérapeutique (dermatologique) ou cosmétique n'avait jamais été à ce jour, mise en évidence.

Sans vouloir être lié à une quelconque théorie, ces molécules ont été testées sur l'activité anti-dégranulante des basophiles humains en partant de l'idée que, l'organisme des mammifères ne possédant pas les enzymes nécessaires à la dégradation des unités galactose associées en α, ces oligosaccharides pouvaient perturber la glycolyse des mastocytes c'est-à-dire la source d'énergie de la granulation et de la dégranulation.

Les oligosaccharides utilisés pour mettre en oeuvre la présente invention comportent de trois à cinq unités osidiques, avec au moins une unité D - galactose liée par son carbone 1 au carbone 6 d'une unité sucrose, au moyen d'une liaison α selon la formule 1 suivante : 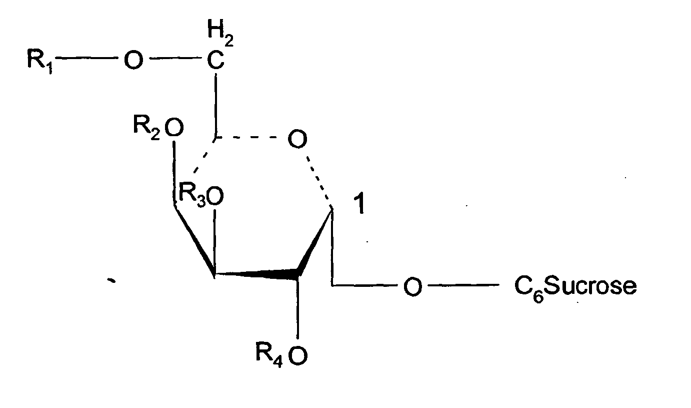 dans laquelle R₁, R₂, R₃ et R₄ représentent indépendamment l'un de l'autre un atome d'hydrogène, un groupe alkyle contenant de 1 à 10 atomes de carbone et possédant éventuellement au moins une insaturation, une fonction acide contenant de 1 à 10 atomes de carbone et possédant éventuellement au moins une insaturation, une fonction sulfate ou encore un ose qui peut se présenter sous forme libre ou dérivée.

En outre, l'unité sucrose peut aussi se présenter sous forme libre (c'est-à-dire un sucre dans lequel les fonctions alcools ne sont pas protégées) ou dérivée. Par ailleurs, cette unité sucrose semble indispensable à l'activité biologique puisque le mélibiose, testé dans les mêmes conditions d'expérience, ne présente strictement aucune activité sur les mastocytes.

Par « forme dérivée » au sens de la présente invention on entend que les fonctions alcools sont substituées par exemple par un groupe acétyle (CH₃CO)...

De manière préférée, on utilisera le stachyose et le raffinose.

Le stachyose est un tétraholoside dont la structure finale est : α-D-Galactopyranosyl-(1-6)-α-D-Galactopyranosyl-(1-6)-α-D-Glucopyranosil-(1-2)-β-D-Fructofuranoside.

Le stachyose et le raffinose correspondent à la formule présentée ci-dessous. 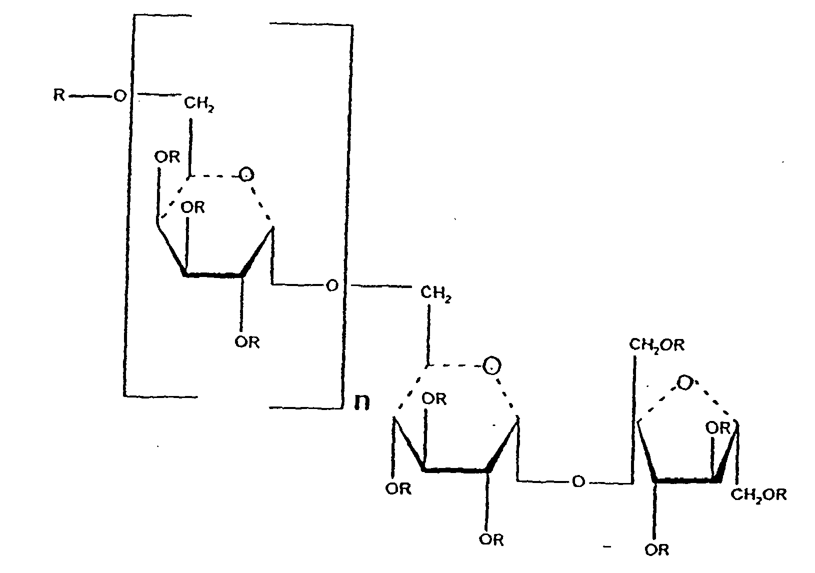 avec pour le stachyose R = H et n = 2 et pour le raffinose R = H et n = 1.

Les compositions, en particulier les composition cosmétiques ou à titre de médicament (dermatologiques), selon l'invention peuvent être présentées sous forme de pommades, crèmes, collyre, sprays ou lotions pour l'administration locale, en association avec des excipients compatibles. Les excipients généralement utilisés pour préparer de telles compositions sont des liants, des conservateurs, des arômes.... Dans ces formes, le pourcentage pondéral en principe actif par rapport à la masse de la composition totale est compris entre 0,01 % et 20 %, de préférence 0,2 et 1 %. Des compositions pharmaceutiques d'inhalation peuvent aussi être préparées pour les traitements internes (asthme).

Afin de tester l'activité anti-dégranulante ou anti-activatrice des oligosaccharides sur les basophiles humains par cytométrie de flux, des suspensions leucocytaires de sujets différents et sélectionnés comme étant de bons répondeurs à l'anti-igE ont été utilisées pour isoler, par sédimentation simple à 1g, les leucocytes. Ceux-ci sont pré-incubés pendant 30 minutes avec des dilutions successives du produit à tester de 10 à 0,01 mg/ml. Les basophiles sont alors stimulés par une concentration cible d'un anti-IgE humain et marqués par un mélange des anticorps anti-IgE FITC, anti-CD63PE, le CD63 étant un marqueur d'activation des basophiles humains. Les pourcentages d'activation ou d'inhibition sont alors calculés par le cytomètre de flux. L'activité biologique des oligosaccharides a été ainsi évaluée.

La structure chimique des molécules commerciales et des dérivés, en particulier celle du stachyose, a été vérifiée par RMN : RMN ¹H et ¹³C, RMN-2D (DQF-COSY, HMQC et HMBC).

Les mêmes tests biologiques ont été effectués sur des analogues structuraux comme le raffinose ou des oligosaccharides comportant une liaison α entre une unité galactose et une autre unité ose comme dans le melibiose.

Ces molécules fortement hydrosolubles n'ont que peu de chance de franchir les membranes liposolubles des mastocytes, ce que confirme les résultats des tests.

Cependant il est bien connu que les mastocytes ont une activité de pinocytose importante vis-à-vis de molécules chargés positivement (des bases donc) au pH physiologique.

Des essais ont donc été entrepris. Ces essais ont pour but de tester le pouvoir d'inhibition de la dégranulation de compositions contenant un premier composant -le principe actif- qui est un oligosaccharide choisi parmi le stachyose et le raffinose à différentes concentrations et un deuxième composant choisi dans le groupe fourni par l'arginine, la choline, le chlorure de calcium (CaCl₂) et la saponine. Le pourcentage d'inhibition a été calculé en renouvelant trois fois l'expérience.

Les résultats obtenus sont consignés dans le tableau 1, les abréviations suivantes ont été utilisées :

| | | | |
|---|---|---|---|
| "S" : | pour Stachyose | R : | pour Raffinose |
| "10" : | pour 10 mg/ml | 1 : | pour 1mg/ml |
| "0,1" : | pour 0,1 mg/ml | "a" | pour arginine |
| "b": | pour choline | "c" | :pour CaCl₂ |
| "Sp" : | pour Saponine | "NT": | pour non testé. |

| Composition | S₁₀a | S₁a | S_{0,1}a | S₁₀b | S₁b | S_{0,1}b |
|---|---|---|---|---|---|---|
| % d'hinibition | 31 | 15 | 0 | 9 | 0 | 0 |

| Composition | S₁₀c | S₁c | S_{0,1}c | S₁₀sp | S₁sp | S_{0,1}sp |
|---|---|---|---|---|---|---|
| % d'inhibition | 6 | 0 | 0 | 76 | 0 | 0 |

| Composition | R₁₀a | R₁a | R_{0,1}a | R₁₀b | R₁b | R_{0,1} b |
|---|---|---|---|---|---|---|
| % d'hinibition | 19 | 5 | 0 | 2 | NT | NT |

| Composition | R₁₀c | R₁c | R_{0,1}c | R₁₀sp | R₁sp | R_{0,1}sp |
|---|---|---|---|---|---|---|
| % d'inhibition | 10 | NT | NT | 77 | 10 | 0 |

Au vu de ces résultats, il apparaît nettement que, aux concentrations testées, les compositions contenant de l'arginine et du stachyose ou du raffinose permettent d'inhiber la dégranulation ou la mise en route du système de granulation et donc d'inhiber la libération d'histamine, de bradykinine, de sérotonine et des facteurs chimio-tactiques d'autres leucocytes.

Afin d'obtenir une plus grande inhibition des mastocytes, nous avons alors entrepris de favoriser la pénétration de ces molécules à travers les membranes en remplaçant l'arginine par de la saponine (Quillaja bark). Les résultats du tableau 1 montrent que des pourcentages d'inhibitions encore plus élevés sont alors obtenus.

L'utilisation des oligosaccharides selon la présente invention, en présence de molécules chargées positivement au pH physiologique (comme par exemple l'arginine) favorisant la pinocytose ou de molécules favorisant la pénétration membranaire (par exemple des saponines) permettent donc de bloquer ou réduire les activités biologiques des mastocytes, basophiles et éosinophiles chez les mammifères.

Aucune activité cytotoxique n'a été constatée au cours des études biologiques. Ce type de molécules peut donc être utilisé avec profit dans tous les domaines thérapeutiques (dermiques) et cosmétiques où les mastocytes et les basophiles humains (et par suite les éosinophiles) sont impliqués. Citons à titre d'exemple le prurit, les piqûres d'insectes, le psoriasis, la conjonctivite allergique, l'urticaire, l'eczéma et l'asthme.

Les exemples qui suivent ne limitent en aucun cas la portée de la présente invention.

### Exemples de formulation :

### Gel topique

| | |
|---|---|
| Oligosaccharide | 0,1 g |
| Arginine (ou saponines) | 0,01 g |
| Méthylcellulose | 3 g |
| Eau purifiée qsp | 100 g |
| Conservateurs, parfum | QS |

### Crème grasse

| | |
|---|---|
| Oligosaccharide | 0,1 g |
| Arginine (ou saponines) | 0,01 g |
| Eau purifiée | 5 ml |
| Corps gras qsp | 100 g |
| Conservateurs, parfum (appliquée telle quelle ou tulle gras) | QS |

### Emulsion

| | |
|---|---|
| Oligosaccharide | 0,2 g |
| Arginine (ou saponines) | 0,02 g |
| Monostéarate de PEG 1500 | 5 g |
| Monostéarate de PEG 300 | 2 g |
| Huile de paraffine fluide | 5 g |
| Eau purifiée qsp | 100 g |
| Conservateurs, parfum | QS |

### Inhalation

Une préparation destinée à l'inhalation est obtenue à partir du mélange suivant :

| | |
|---|---|
| Oligosaccharide | 100 mg |
| Arginine (ou saponines) | 10 mg |
| Excipient | 10 g |

A ce mélange, on ajoute un excipient classique pour formulation pour inhalation, comme par exemple l'acide oléique, ainsi qu'un gaz propulseur de type trichlorofluorométhane, dichlorodifluorométhane.

### Crème grasse A (formule centésimale)

| Composants | % |
|---|---|
| Eau | 54,60 |
| PEG-6 et PEG-32 stéarate | 12,00 |
| Butyrospermum parkii | 10,00 |
| Raffinose | 10,00 |
| Glycérine | 7,00 |
| Paraffinum liquidum | 3,00 |
| Acide stéarique | 1,20 |
| Saponine | 1,00 |
| Méthylparaben | 0,20 |
| Imidazolidinyl urée | 0,20 |
| Bisabolol | 0,20 |
| Parfum | 0,20 |
| Propylparaben | 0,10 |
| BHT, BHA, propylgallate et acide citrique | 0,05 |
| Alcool benzylique, méthylchloroisothiazolinone et méthylisothiazolinone | 0,05 |

### Effet antiprurigineux sur le tégument récemment épidermisé du grand brûlé

En phase de cicatrisation, les grands brûlés souffrent de démangeaisons intenses, même parfois sous traitement lourd d'anti- histaminiques.

Cette étude, d'une durée d'un an, portant sur 33 patients a mis en évidence l'effet antiprurigineux puissant de la composition A dans cette indication.

### PROTOCOLE

Les patients souffrant de démangeaisons sont traités par une application de la composition A dont la concentration en raffinose est de 10%, ou par une application de la composition A' dont la concentration en raffinose est de 5%, ou encore par une application de la composition A" dont la concentration en raffinose est de 3% l'application étant attribuée par tirage au sort. Le pourcentage en saponine des compositions A' et A" représente un dixième de la concentration en raffinose.
En cas de deuxième zone à traiter parallèlement, le patient se voit appliquer en double aveugle et à son insu une composition considérée comme placebo sur cette deuxième zone contenant les mêmes excipients mais ne contenant ni raffinose, ni saponine.
Les trois tubes concentrés différemment en raffinose sont codés pour le patient et le personnel chargé de l'application.
Les applications sont renouvelées plusieurs fois par jour en cas de démangeaison et à la demande du patient et peuvent s'étaler sur plusieurs jours
Une fiche de suivi indique le temps d'action du ou des produits selon une échelle analogique du prurit de 0 à 10.

### RESULTATS ET CONCLUSIONS

Sur 33 cas traités, 30 ont mis en évidence une action du produit, 3 se sont révélés négatifs.
8 cas ont concerné deux zones d'application contre placebo.
Le temps moyen d'action de la composition A sur les 30 cas positifs se situe entre 2 et 5 mn, avec une durée d'action moyenne comprise entre 3 et 4 heures.
L'intensité du prurit initial estimé en moyenne à 7 sur l'échelle analogique, est à 0 dans les 30 cas après 15 mn.
Aucune différence statistiquement significative n'a pu être mis en évidence quant aux trois dosages en raffinose.
Contre placebo, on observe toujours les mêmes effets que ci-dessus pour la zone traitée avec la composition A.
Pour la zone traitée avec le placebo, l'intensité du prurit baisse légèrement de 0 à 5 mn pour remonter au niveau de l'intensité initiale au bout de 5 à 15mn. Ce phénomène transitoire bien connu est lié à l'hydratation de la peau.
Aucun phénomène d'intolérance n'a été signalé lors de l'étude en application simple ou répétée.
A signaler qu'un cas d'eczéma a régressé sous l'effet du traitement
Sur les 3 cas négatifs, l'un est inexpliqué et les deux autres patients souffraient, pour l'un, de très forte inflammation et pour l'autre de sensation de cuisson, mais pas réellement de prurit.

## Revendications

1. Composition contenant une association d'au moins deux composants, et éventuellement un excipient, destinée à être administrée par voie topique externe ou par inhalation, telle que le premier composant est un oligosaccharide de formule I contenant de trois à cinq unités osidiques et possédant au moins une unité D-galactose liée par une liaison α[1-6] avec une unité sucrose : 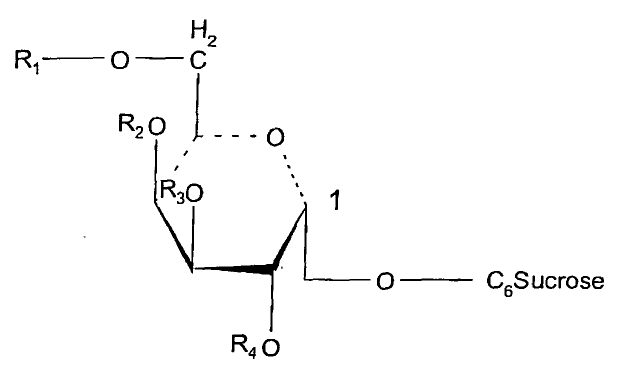 dans laquelle R₁, R₂, R₃ et R₄, indépendamment les uns des autres représentent un atome d'hydrogène, un groupe alkyle contenant de 1 à 10 atomes de carbone et possédant éventuellement au moins une insaturation, une fonction acide contenant de 1 à 10 atomes de carbone et possédant éventuellement au moins une insaturation, une fonction sulfate ou encore un ose et
le deuxième composant est une saponine ou un acide aminé basique, la concentration du deuxième composant étant comprise entre 5 % et 20 % en poids par rapport à la concentration en oligosaccharides.

2. Composition selon la revendication 1, telle que ledit oligosaccharide est choisi dans le groupe constitué par le raffinose et le stachyose sous forme libre ou dérivée.

3. Composition selon la revendication 1 ou 2, telle que le deuxième composant est choisi dans le groupe constitué par les saponines et l'arginine.

4. Composition selon l'une des revendications 1 à 3, telle que l'oligosaccharide est le raffinose sous forme libre ou dérivée et le deuxième composant est une saponine ou l'arginine.

5. Composition selon l'une des revendications 1 à 3, telle que l'oligosaccharide est le stachyose sous forme libre ou dérivée et le deuxième composant est une saponine ou l'arginine.

6. Composition selon l'une des revendications précédentes **caractérisée en ce que** la concentration pondérale en oligosaccharides est comprise entre 0,01 % et 20 % par rapport à la masse totale de la composition.

7. Composition selon l'une des revendications précédentes **caractérisée en ce que** la concentration pondérale en oligosaccharides est comprise entre 0,01 % et 10% par rapport à la masse totale de la composition et la concentration du deuxième composant est environ 10 % en poids par rapport à la concentration en oligosaccharides.

8. Composition selon l'une des revendications précédentes telle qu'elle se présente sous la forme d'une pommade, d'une crème, d'un collyre, d'une lotion, d'un spray destiné à la voie externe ou d'un spray pour inhalation.

9. Composition selon l'une des revendications précédentes telle qu'il s'agit d'une composition cosmétique, dermatologique ou d'un médicament.

## Claims

1. Composition containing a combination of at least two components, and optionally an excipient, which composition is intended to be administered topically and externally or by inhalation, such that the first component is an oligosaccharide of formula I containing from three to five osidic units and having at least one D-galactose unit linked via an α[1-6] bond with a sucrose unit: 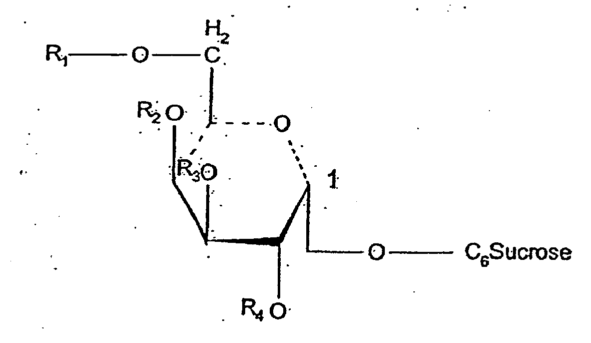 in which R₁, R₂, R₃ and R₄, independently of each other, represent a hydrogen atom, an alkyl group containing from 1 to 10 carbon atoms and optionally having at least one unsaturation, an acid function containing from 1 to 10 carbon atoms and optionally having at least one unsaturation, a sulphate function or a saccharide, and
the second component is a saponin or a basic amino acid, the concentration of the second component being between 5% and 20% by weight relative to the oligosaccharide concentration.

2. Composition according to Claim 1, such that the said oligosaccharide is chosen from the group consisting of raffinose and stachyose in free or derived form.

3. Composition according to Claim 1 or 2, such that the second component is chosen from the group consisting of saponins and arginine.

4. Composition according to one of Claims 1 to 3, such that the oligosaccharide is raffinose in free or derived form and the second component is a saponin or arginine.

5. Composition according to one of Claims 1 to 3, such that the oligosaccharide is stachyose in free or derived form and the second component is a saponin or arginine.

6. Composition according to one of the preceding claims, **characterized in that** the weight concentration of oligosaccharides is between 0.01% and 20% relative to the total mass of the composition.

7. Composition according to one of the preceding claims, **characterized in that** the weight concentration of oligosaccharides is between 0.01% and 10% relative to the total mass of the composition and the concentration of the second component is about 10% by weight relative to the oligosaccharide concentration.

8. Composition according to one of the preceding claims, such that it is in the form of an ointment, a cream, eyedrops, a lotion, a spray intended for external use, or a spray for inhalation.

9. Composition according to one of the preceding claims, such that it is a cosmetic or dermatological composition, or a medicament.

## Patentansprüche

1. Zusammensetzung, enthaltend eine Assoziation von mindestens zwei Verbindungen und gegebenenfalls einen Hilfsstoff, die dazu bestimmt ist, auf äußerem topischem Weg oder durch Inhalation verabreicht zu werden, derart, dass die erste Verbindung ein Oligosaccharid der Formel I ist, das drei bis fünf osidische Einheiten enthält und mindestens eine D-Galactose-Einheit besitzt, die durch eine a[1-6]-Bindung mit einer Saccharose-Einheit verbunden ist: 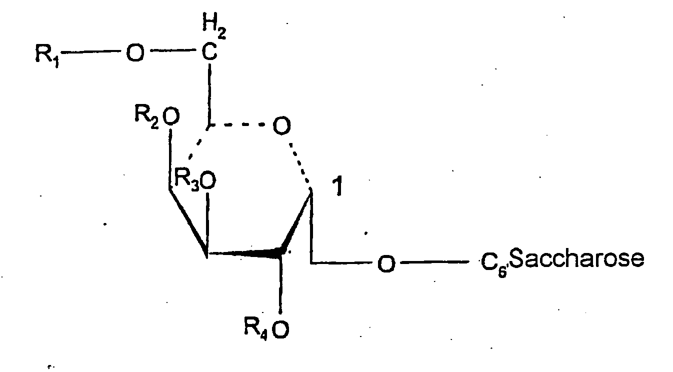 worin R₁, R₂, R₃ und R₄ unabhängig voneinander ein Wasserstoffatom, eine Alkylgruppe, die 1 bis 10 Kohlenstoffatome enthält und gegebenenfalls mindestens eine Unsättigung aufweist, eine Säurefunktion, die 1 bis 10 Kohlenstoffatome enthält und gegebenenfalls mindestens eine Unsättigung aufweist, eine Sulfatfunktion oder auch eine Ose sind, und
die zweite Verbindung ein Saponin oder eine basische Aminosäure ist, wobei die Konzentration der zweiten Verbindung zwischen 5 und 20 Gew.-% einschließlich, bezogen auf die Konzentration an Oligosacchariden, beträgt.

2. Zusammensetzung nach Anspruch 1, derart, dass das Oligosaccharid aus der Gruppe ausgewählt ist, die aus Raffinose und Stachyose in freier oder derivatisierter Form besteht.

3. Zusammensetzung nach Anspruch 1 oder 2, derart, dass die zweite Verbindung aus der Gruppe ausgewählt ist, die aus Saponinen und Arginin besteht.

4. Zusammensetzung nach einem der Ansprüche 1 bis 3, derart, dass das Oligosaccharid Raffinose in freier oder derivatisierter Form ist und die zweite Verbindung ein Saponin oder Arginin ist.

5. Zusammensetzung nach einem der Ansprüche 1 bis 3, **dadurch gekennzeichnet, dass** das Oligosaccharid Stachyose in freier oder derivatisierter Form ist und die zweite Verbindung ein Saponin oder Arginin ist.

6. Zusammensetzung nach einem der vorangehenden Ansprüche, **dadurch gekennzeichnet, dass** die Gewichtskonzentration an Oligosacchariden zwischen 0,01 % bis 20% einschließlich, bezogen auf das Gesamtgewicht der Zusammensetzung, beträgt.

7. Zusammensetzung nach einem der vorangehenden Ansprüche, **dadurch gekennzeichnet, dass** die Gewichtskonzentration an Oligosacchariden 0,01% bis 10%, bezogen auf das Gesamtgewicht der Zusammensetzung, beträgt und die Konzentration der zweiten Verbindung etwa 10 Gewichts-%, bezogen auf die Konzentration an Oligosacchariden, beträgt.

8. Zusammensetzung nach einem der vorangehenden Ansprüche, derart, dass die Zusammensetzung in Form einer Salbe, einer Creme, von Augentropfen, einer Lotion, eines Sprays, das für den äußeren Weg bestimmt ist, oder eines Sprays zur Inhalation vorliegt.

9. Zusammensetzung nach einem der vorangehenden Ansprüche, derart, dass es sich um eine kosmetische, dermatologische Zusammensetzung oder um ein Medikament handelt.
